Europäisches Patentamt

(19) European Patent Office

Office européen des brevets

(11) Numéro de publication: 0 018 917
A1

## (12) DEMANDE DE BREVET EUROPEEN

(21) Numéro de dépôt: 80400606.2

(22) Date de dépôt: 06.05.80

(51) Int. Cl.³: C 12 M 1/00

(30) Priorité: 08.05.79 FR 7911956

(43) Date de publication de la demande:
12.11.80 Bulletin 80/23

(84) Etats Contractants Désignés:
AT CH DE GB IT LI NL

(71) Demandeur: SOCIETE D'ETUDES DE TECHNIQUES ET
DE REALISATIONS INDUSTRIELLES ET
COMMERCIALES (SETRIC)
Zone Industrielle de Montaudran Boîte Postale 4050
Avenue Didier Daurat F-31027 Toulouse Cédex(FR)

(72) Inventeur: Chelle, René
11, rue Maran
F-31400 Toulouse(FR)

(72) Inventeur: Sanchez, Christian
6, "La Gentille"De Salvetat-Saint-Gilles
F-31770 Tournefeuille(FR)

(74) Mandataire: Fruchard, Guy et al,
Cabinet Chereau & Cabinet Rodes Réunis 107, boulevard
Pereire
F-75017 Paris(FR)

(54) Procédé d'aération, d'évacuation des gaz de fermentation et d'élimination de calories dans les cultures de micro-organismes en milieu solide et dispositifs permettant de le mettre en oeuvre.

(57) Un tel procédé consiste à créer, à l'intérieur du milieu de culture, au moins un cycle de variation de pression pour autoriser pendant la phase de pressurisation la pénétration de l'agent réactif en tous les points du milieu et pendant la phase de dépressurisation l'évacuation des gaz de fermentation et l'élimination d'un partie des calories dégagées par la réaction exothermique du métabolisme microbien, grâce à l'extraction de la vapeur d'eau.

L'invention concerne également des dispositifs permettant de metre en oeuvre un tel procédé.

Fig.1

Croydon Printing Company Ltd.

0018917

# PROCEDE D'AERATION, D'EVACUATION DES GAZ DE FERMENTATION ET D'ELIMINATION DE CALORIES DANS LES CULTURES DE MICRO-ORGANISMES EN MILIEU SOLIDE ET DISPOSITIFS PERMETTANT DE LE METTRE EN OEUVRE.

L'invention concerne un procédé applicable aux cultures de micro-organismes en milieu solide et qui permet, à la fois, l'oxygénation des micro-organismes, l'élimination des gaz de fermentation et tout ou partie des calories produites par le métabolisme microbien ; l'invention s'étend aux dispositifs de fermentation pour la mise en oeuvre du procédé.

On peut distinguer plusieurs procédés de cultures pour les micro-organismes : culture en milieu liquide stationnaire, culture en milieu liquide agité, culture en milieu solide.

Dans le cas de culture en milieu liquide stationnaire, les micro-organismes aérobies surnagent le plus souvent en surface du bouillon liquide. Cette technique ne trouve d'applications que dans des cas bien particuliers mettant en jeu de très faibles volumes de milieu de culture. Chaque module de réacteur de culture ne dépasse pas la taille d'un appareil de laboratoire.

Dans le cas de culture en milieu liquide agité, les micro-organismes sont immergés dans le bouillon de culture. L'oxygène qui leur est nécessaire est apporté par une injection d'air au sein du liquide. Cet oxygène doit passer de la phase gazeuse à la phase liquide, c'est à dire, doit être dissous dans le liquide avant d'être assimilé par les cellules. La dissolution de l'oxygène est coûteuse en énergie car une faible partie seulement de l'oxygène injecté est dissoute et parce que les vitesses de dissolution satisfaisantes ne sont obtenues qu'au prix d'une agitation mécanique du milieu de culture (procédé classique) ou d'un brassage important assuré par une injection massive d'air comprimé ou surpressé (procédé air lift).

Dans le cas de culture en milieu solide, les micro-organismes aérobies se développent en surface de particules solides imbibées d'eau et de substances nutritives, ou sur la surface interne des espaces interstitiels d'un milieu poreux. l'air doit cir-

0018917

ler entre les particules pour apporter aux micro-organismes l'oxygène qui leur est nécessaire. Contrairement aux cultures en milieu liquide agité, l'oxygène diffuse directement depuis la phase gazeuse jusqu'aux cellules sans avoir à être dissous dans un liquide intermédiaire. On conçoit donc que cette technique conduise à une économie d'énergie par rapport aux techniques classiques de fermentation. Cette économie représente l'un des nombreux avantages des cultures en milieu solide par rapport aux techniques classiques de fermentation. On peut citer comme autres avantages : une concentration plus élevées en substrat, une teneur plus faible en eau, une productivité des réacteurs plus élevée.

Les cultures en milieu solide n'ont cependant pas trouvé d'applications industrielles en dehors de cas bien particuliers et depuis longtemps abandonnés tels que la culture sur plateaux en couches minces. Elles ne sont pas développées pour une raison essentielle : sur de petits volumes, au stade laboratoire, les particules sont disposées en amas de faible épaisseur. Cette épaisseur est suffisamment faible pour que l'air puisse circuler ou diffuser librement depuis la périphérie jusqu'au coeur des amas. Sur de gros volumes, à l'échelle industrielle, au contraire, les amas ont une taille telle que l'oxygène ne peut pas diffuser suffisamment vite de la périphérie vers le centre. On est donc obligé de réaliser une circulation forcée d'air à travers ces amas de particules. Or, lorsqu'on injecte de l'air en un ou plusieurs points de ces amas, l'air chemine en continu et emprunte, dans la plupart des cas, des passages préférentiels. Les micro-organismes aérobies se développent donc sur les particules situées dans les zones de passages préférentiels, ne se développent pas dans les autres zones qui sont soumises à des conditions d'anaérobiose. Pour supprimer l'influence néfaste de ces passages préférentiels, certains procédés consistent à agiter mécaniquement toute la masse des particules : fermenteurs en tambours tournants, par exemple. Dans la plupart des cas, cette agitation, ou bien est insuffisante, ou bien conduit à une agglomération des particules entre elles et la culture aérobie est rendue impossible.

En résumé, les techniques de culture en milieu solide ne sont pas passées du stade de laboratoire au stade industriel, car elles se sont heurtées à un problème essentiel : l'aération et le maintien en aérobie des micro-organismes en tous points de la masse de ce milieu. Les tentatives de cultures en plateaux sur couches

001891

minces ont été abandonnées car elles exigent des manipulations lourdes et le maintien en asepsie est trop délicat ; les fermenteurs en milieu solide avec agitation mécanique ne sont pas développés car, dans la plupart des cas, les particules ont tendance à former des agglomérats de grande taille impénétrables à l'oxygène.

Le procédé, selon l'invention, a pour objet d'assurer les échanges gazeux en tous les points de la masse du milieu solide quel que soit son volume et sans qu'il soit nécessaire de l'agite mécaniquement. Le procédé consiste à appliquer dans la masse de milieu solide des cycles de pressurisation et de dépressurisation de façon, d'une part, à vaincre la résistance mécanique qui s'oppose à la pénétration et à la diffusion des gaz en tous les point. du milieu et, d'autre part, à évacuer le mélange gazeux riche en $CO_2$ de tous les espaces interstitiels du milieu de culture. Les cycles de pressurisation et de dépressurisation peuvent être obtenus par des moyens appropriés tels que la pressurisation par de compresseurs ou surpresseurs d'air et dépressurisation jusqu'à la pression atmosphérique par ouverture de la sortie d'air ou bie tels que dépressurisation par pompe à vide et remise à la pressio atmosphérique par admission d'air atmosphérique. Dans tous les cas, l'air admis dans le réacteur est de l'air stérile. Le procédé est donc caractérisé par la succession des deux phases :

Phase 1 : pressurisation progressive jusqu'à la pression maximale P1 et maintien éventuel à cette pression P1.

Phase 2 : dépressurisation progressive jusqu'à la pression minimale P2 et maintien éventuel à cette pression P2.
La pression P1 peut être la pression atmosphérique ou une pression supérieure. La pression P2 peut être une dépression en dessous de la pression atmosphérique ou la pression atmosphérique elle-même.

Durant la première phase (de montée en pression), l'air ou le gaz injecté envahit d'abord les passages préférentiels. Il se crée alors un gradient de pression entre les zones de passages préférentiels (haute pression) et les autres zones (basse pression). Tout au long de cette première phase, le gradient de pression disparaît progressivement dans le réacteur car l'air en émigrant des zones de haute pression vers les zones de basse pression, envahit toute la masse du milieu du solide. Au bout d'un temps suffisant, on peut obtenir une pression homogène dans tout le réacteur. Les échanges gazeux peuvent alors se produire en tous points du milieu. Les gaz de fermentation se mélangent à l'air injecté et l'oxygène diffuse dans les cellules qui tapissent les espaces interparticulaires ou les pores. De plus, la pressurisation a pour conséquence d'augmenter la pression partielle en air riche en oxygène et pauvre en gaz carbonique dans l'atmosphère des espaces interparticulaires qui, elle, est riche en gaz carbonique et pauvre en oxygène. Ainsi, les échanges gazeux sont favorisés et accélérés. La migration du gaz injecté à travers toute la masse du milieu peut être plus ou moins rapide suivant le degré de vide et les pertes de charge à vaincre. Il convient donc de régler la durée de la première phase de pressurisation et de maintien à la pression maximale P1 pour que le gaz injecté atteigne bien tous les points de la masse de milieu.

Au cours de la deuxième phase ( de dépressurisation et de maintien à la pression minimale P2), le phénomène inverse se produit : le mélange air injecté-gaz de fermentation sort du réacteur. La pression chute rapidement dans certaines zones, tandis qu'elle chute moins rapidement dans les espaces interparticulaires les moins accessibles. Il s'établit donc un gradient de pression entre ces différentes zones. Ce gradient va s'annuler progressivement car les gaz émigrent depuis les points les moins accessibles jusqu'à la sortie du réacteur. La durée de l'évacuation dépend des pertes de charge à vaincre pour traverser la culture. Il convient donc de régler la durée de dépressurisation et de maintien à la pression minimale P2 en fonction du type de culture et de milieu.

L'amplitude et la fréquence du cyle pressurisation-dépressurisation doivent être réglées selon le type de culture et de milieu ; l'amplitude pouvant varier de 0,1 à 10 bars et la fréquence pouvant être de l'ordre de 1 minute à plusieurs heures comme on le verra dans les exemples décrits ultérieurement.

Le procédé, selon l'invention, permet donc de résoudre le problème posé par les phénomènes de passages préférentiels. Le cycle pressurisation-dépressurisation oblige en effet le gaz injecté à se repartir d'une façon homogène en tous les points de la masse du milieu quels que soient la résistance mécanique à la pénétration et le volume du réacteur.

Le procédé permet donc d'apporter aux micro-organismes aérobies l'oxygène dont ils ont besoin et ceci quelle que soit leur position au sein du réacteur. Il permet, en outre, de réaliser un

5

0018917

mélange entre les gaz injectés et les gaz de fermentation dans tous les espaces interparticulaires ou tous les pores du milieu solide, et par voie de conséquence, d'entraîner et d'évacuer, avec l'air, les gaz de fermentation.

Il est important de noter que certains gaz de fermentation sont inhibiteurs et que leur évacuation améliore la vitesse de croissance des micro-organismes. Comme les autres gaz, la vapeur d'eau qui se trouve dans les interstices est éliminée par l'air, ce qui entraîne une nouvelle évaporation dans ces interstices ; cette évaporation permet d'éliminer des calories. Il convient donc, si l'évaporation est importante, d'ajouter la quantité d'eau liquide nécessaire à maintenir le taux d'humidité du milieu constant. Le procédé a donc pour conséquence l'évacuation de calories dans toute la masse du milieu. Toutefois, les débits de gaz mis en jeu pour obtenir une bonne aération ou une bonne évacuation des gaz de fermentation peuvent être insuffisants pour évacuer toutes les calories provenant des réactions métaboliques exothermiques. Il faut alors compléter le système de fermentation par un dispositif complémentaire. Ce dispositif peut être un échangeur de chaleur convenablement disposé à l'intérieur de la masse, qui vient augmenter la surface d'échange constituée par les parois du réacteur elles-mêmes.

Les moyens pour mettre en oeuvre le procédé peuvent être différents suivant le cycle de pression que l'on souhaite obtenir et qui est le plus approprié au type de culture réalisé.

L'invention s'étend donc aux dispositifs de fermentation qui permettent la mise en oeuvre du procédé et, de façon générale, à tous les dispositifs qui peuvent permettre de créer à l'intérieur d'une culture en milieu solide un cycle de pressurisation-dépressurisation au moyen d'air ou d'un autre gaz.

Ces dispositifs peuvent être des dispositifs de culture discontinue ou des dispositifs de culture continue. Dans ce dernier cas, on introduit du milieu solide en continu à l'entrée du réacteur et on soutire, à la sortie, la quantité correspondante de milieu fermenté.

EXEMPLE I

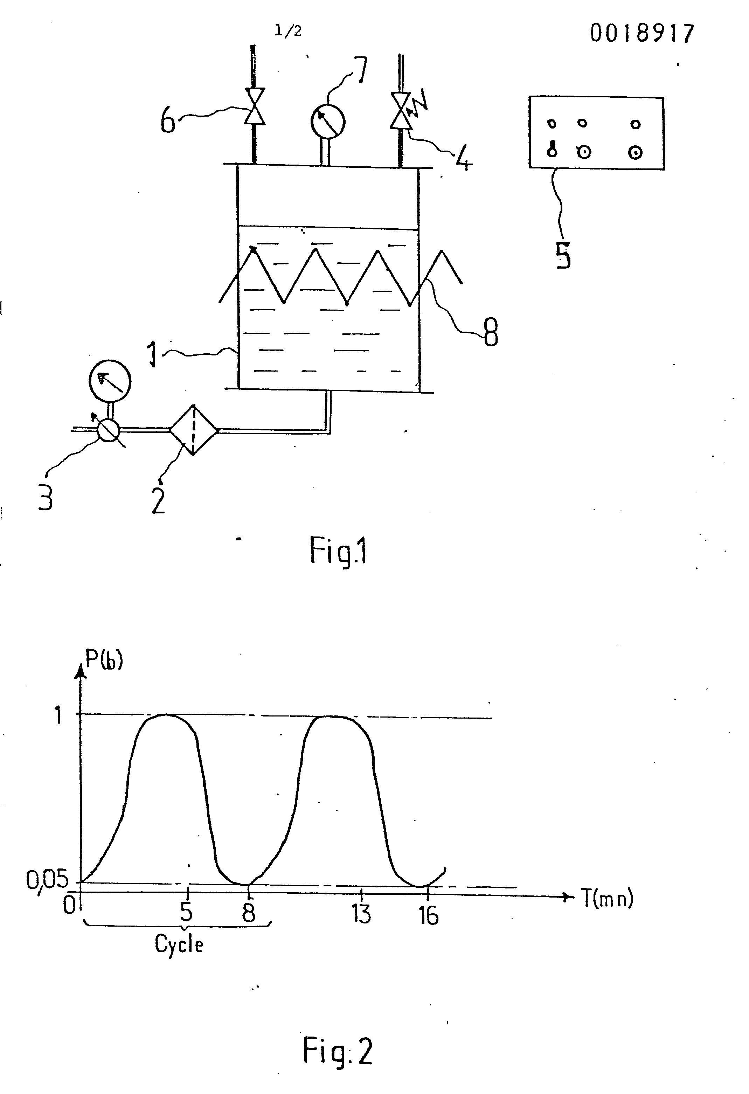

La figure 1 représente, à titre d'exemple non limitatif, l'un des dispositifs que l'on peut utiliser pour mettre en oeuvre le procédé. Ce dispositif permet d'obtenir des cycles de pressurisation-dépressurisation tels que celui représenté sur la courbe de la figure 2.

Selon la figure 1, on dispose un fermenteur 1 contenant le milieu solide. A la base du fermenteur arrive de l'air stérile filtré sur le filtre bactériologique 2. La pression de l'air entrant dans le réacteur ne peut pas dépasser un seuil imposé par le manodétendeur 3. L'air qui a traversé le fermenteur est évacué à travers l'électrovanne 4. Le cycle d'ouverture et de fermeture de cette électrovanne 4 est commandé par le programmateur 5. Un tube 6 permet d'injecter, dans le réacteur, l'inoculum et tout additif nécessaire à la culture. Les manomètres de contrôle 3 et 7 permettent de suivre les variations de pression à l'entrée et à la sortie du réacteur. Un échangeur de chaleur 8 permet d'assurer une certaine constance de température au sein de la masse.

Pour obtenir le cycle de pression décrit par la courbe de la figure 2, la manodétendeur est réglé à une pression relative maximum de 1 bar ; il laisse donc passer de l'air tant que la pression dans le fermenteur n'a pas atteint 1 bar. L'électrovanne 4 est maintenue fermée pendant 5 minutes, puis ouverte pendant 3 minutes. Ce dispositif et ce cycle de pression ont permis d'obtenir les résultats décrits ci-après. Ces résultats permettent de comparer l'efficacité de l'aération selon le procédé, objet de l'invention, et selon le procédé d'injection continue d'air.

Dans le premier cas, on applique le cycle de pression de la figure 2 grâce au dispositif de la figure 1 où le volume du fermenteur est de 2 litres.

Dans le deuxième cas, on injecte en continu de l'air dans ce même fermenteur de 2 litres mais sans appliquer de cycle de variation de pression. Le support solide est ici constitué de sciure de liège (60g) imbibée par 200ml de milieu ayant la composition suivante :

- Mélassé................ 40 g/l
- $PO_4(NH_4)H_2$......... 5 g/l
- $SO_4(NH_4)_2$.......... 5 g/l
- $MgSO_4, 7H_2O$........ 0,2 g/l
- Corn Steep atomisé.... 2 g/l

Le milieu dont le pH est ajusté à 7, est mélangé à la sciure. Le support imbibé de milieu est introduit dans le fermenteur. Le fermenteur, muni de son filtre à air, est porté à l'autoclave 30 minutes à 120°C. Après stérilisation il est placé dans un bain thermostaté à 30°C et le cycle de variation de pression d'air

est mis en route dans le premier cas, tandis que l'air est injecté en continu dans le deuxième cas. Dans les deux cas, le débit d'air moyen est de 0,5 vvm. Le milieu est ensemencé aseptiquement par une suspension de levures (Saccharoyces cerevisiae). Après 48 heures de culture, on rajoute 250 ml du milieu défini ci-dessus ; les résultats de croissance sont reportés dans le tableau ci-après :

| Résultats obtenus selon le procédé | | | Résultats obtenus par injection continue d'air | |
|---|---|---|---|---|
| Durée de culture | Levures/g de substance humide | Levures/g de substance sèche | Levures/g de substance humide | levures/g de substance sèche |
| 0 | $5\text{-}10^6$ | | $5\text{-}10^6$ | |
| 72h | Surf.$9.10^8$ Milieu $8.10^8$ Fond $9.10^8$ | $5,2\text{-}10^9$ $4,6\text{-}10^9$ $5,2\text{-}10^9$ | $1,12\text{-}10^8$ | $6,6\text{-}10^8$ |
| 144h | | | $2,06\text{-}10^8$ | $1,5\text{-}10^9$ |

On constate, d'après les résultats, que le procédé permet d'obtenir une quantité de levures bien supérieure à celle que l'on obtient par injection continue d'air. La concentration de levures est homogène dans la culture réalisée selon le procédé. Le nombre de levures plus faible obtenu par injection continue d'air s'explique par une répartition non homogène des levures qui se sont seulement multipliées dans les zones de passages préférentiels. De plus, on note que la vitesse de croissance est supérieure lorsque l'injecd'air est réalisée selon le procédé.

Ce même dispositif de la figure 1 peut être utilisé pour d'autres types de culture, comme par exemple la culture d'un champignon Fusarium graminearum. L'objectif de la culture est la production de zéaralénone, métabolite synthétisé par cette souche de Fusarium. Le milieu de culture est constitué par du riz imbibé d'eau. Le cycle de pressurisation-dépressurisation appliqué au fermenteur est celui représenté sur la figure 2 pendant la phase de croissance (1 semaine à 27°C). Pendant cette phase, le débit d'air moyen est maintenu à 0,5 vvm. Ensuite, la culture est maintenue à 14°C pendant 3 semaines. Au cours de ce maintien à basse température, le débit d'air moyen est ramené à 0,1 vvm. On peut constater un développement normal de la souche, tant du point de vue macroscopique que du point de vue microscopique.

L essai est réalisé en parallèle sur une couche mince de substrat (1 à 2 cm) en fiole d'erlenmeyer de 1 litre bouchée par du coton, technique classique utilisée en laboratoire pour la production de zéaralenone.

On obtient dans les deux cas, des valeurs voisines : 5,5 g de zéaralénone produite par kilogramme de substrat sec. Ce résultat montre que l'on peut cultiver un champignon selon le procédé, sur des couches épaisses de substrat solide et que l'on obtient des résultats analogues à ceux que l'on aurait obtenus selon la technique de culture en couches minces sur plateau.

De plus, un essai a été réalisé dans le réacteur de 2 litres avec injection continue d'air sans appliquer le procédé, c'est à dire sans cycle de pressurisation-dépressurisation ; le débit d'air injecté à la base du réacteur est de 0,5 vvm, cet air ressort par le haut du fermenteur. Après une semaine de culture à 27°C, on peut constater qu'une très faible partie de la masse de milieu est envahie par le feutrage mycélien du Fusarium, tandis que dans l'autre partie, on constate qu'il n'y a pas de développement. En effet, le champignon ne se développe que dans certaines zones de passage préférentiel où circule l'air. On peut donc conclure que le procédé permet aux champignons de se développer dans tous les points de la masse de milieu solide.

EXEMPLE II

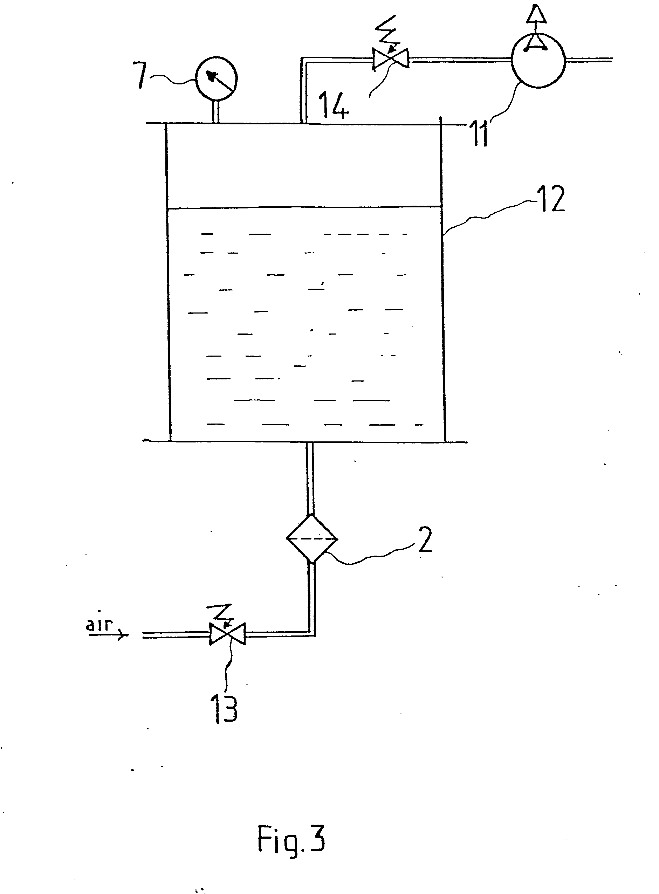

La figure 3 représente un dispositif différent de la figure 1, du fait que le cycle de pression est obtenu en faisant fonctionner un appareil de vide 11 qui crée le vide dans le fermenteur 12 tandis que l'électrovanne 13 est fermée. Lorsque le vide désiré est obtenu et que le mélange air plus gaz de fermentation est évacué, l'électrovanne 14 se ferme tandis que l'électrovanne 13 s'ouvre. A ce moment-là, de l'air stérilisé par filtration est admis dans le fermenteur et la pression remonte à la pression atmosphérique. Pendant cette phase de remontée en pression, l'air se répand en tous points du milieu solide dans la mesure où la résistance à la pénétration jusqu'à ces divers points ne dépasse pas la valeur de la dépression relative obtenue durant la phase précédente.

001891

R E V E N D I C A T I O N S

1. Procédé d'aération, d'évacuation des gaz de fermentation et d'élimination de calories dans les cultures de micro-organismes en milieu solide permettant d'assurer ou d'accélerer la croissance de ces micro-organismes, CARACTERISE EN CE QU'il consiste à créer à l'intérieur du milieu de culture au moins un cycle de variation de pression pour autoriser pendant la phase de pressurisation la pénétration d'un fluide gazeux en tous les points du milieu et pendant la phase de dépressurisation l'évacuation des gaz de fermentation et l'élimination d'une partie des calories dégagées par la réaction exothermique du métabolisme microbien, grâce à l'extraction de la vapeur d'eau.

2. Procédé selon la revendication 1, CARACTERISE EN CE QUE l'amplitude, la durée et le nombre des cycles de variation de pression sont déterminés suivant les besoins de la culture, autrement dit de l'oxygénation, de l'évacuation des gaz de fermentation et de l'élimination des calories.

3. Procédé selon les revendications 1 et 2, CARACTERISE EN CE QUE les variations de pression sont séparées par des paliers de pression dont le niveau et la durée sont réglés en fonction des caractéristiques de la culture et plus particulièrement en fonction de la résistance mécanique qui s'oppose à la pénétration et/ ou à l'évacuation des gaz.

4. Procédé selon les revendications 1 et 2, CARACTERISE EN CE QUE l'amplitude des variations de pression est comprise entre 0,1 et 10 bars suivant les caractéristiques de la culture et de préférence entre 0,5 et 2 bars.

5. Procédé selon les revendications 1, 2 et 3, CARACTERISE EN CE QUE la durée du cycle de variation de pression est comprise entre quelques minutes et plusieurs heures suivant les caracté-ristiques de la culture et de préférence entre 1 minute et 1 heure.

6. Procédé selon l'une quelconque des revendications 1 à 5, CARACTERISE EN CE QUE l'eau évaporée et éliminée pendant la phase de dépressurisation est remplacée périodiquement par l'apport d'une même quantité d'eau sous forme liquide.

7. Procédé selon l'une quelconque des revendications 1 à 6, CARACTERISE EN CE QUE le cycle de variation de pression est obtenu par une injection dans le milieu de culture d'air sous pression qui apporte aux micro-organismes aérobies l'oxygène dont

10

0018917

ils ont besoin et par une évacuation d'air qui entraîne les gaz de fermentation et la vapeur d'eau.

8. Procédé selon l'une quelconque des revendications 1 à 6, CARACTERISE EN CE QUE le cycle de variation de pression est obtenu en faisant le vide dans le milieu de culture par pompage de l'air, des gaz de fermentation et éventuellement de la vapeur d'eau, et en introduisant ensuite de l'air sous la pression atmosphérique pour apporter aux micro-organismes aérobies l'oxygène dont ils ont besoin.

9. Dispositif de fermentation permettant de mettre en oeuvre le procédé selon l'une quelconque des revendications 1 à 8, CARACTERISE EN CE QU'il comprend au moins un conteneur dans lequel est placé le milieu de culture, des moyens d'admission d'air pour pressuriser l'intérieur dudit conteneur et des moyens d'évacuation d'air pour le dépressuriser, lesdits moyens étant associés à une commande séquentielle pour pressuriser et dépressuriser alternativement l'intérieur du conteneur.

10. Dispositif de fermentation selon la revendication 9, CARACTERISE EN CE QUE le maintien de la température du milieu de culture est assuré par un échangeur de chaleur convenablement agencé à l'intérieur dudit milieu, cet échangeur venant s'ajouter à celui constitué par les parois du conteneur.

11. Dispositif de fermentation selon la revendication 9, CARACTERISE EN CE QUE lesdits moyens d'admission d'air sont constitués par un compresseur d'air réglé par un manodétendeur pour obtenir une pression maximum à l'intérieur du conteneur alors que lesdits moyens d'évacuation sont constitués par une vanne disposée sur le circuit de sortie des gaz et assujettie à une commande électrique réglant la durée et le degré de son ouverture.

12. Dispositif de fermentation selon la revendication 9, CARACTERISE EN CE QUE lesdits moyens d'évacuation des gaz sont constitués par un appareil à vide réglé par une vanne disposée sur le circuit d'aspiration des gaz et assujettie à une commande électrique limitant la durée et le degré de son ouverture alors que lesdits moyens d'admission sont constitués par une vanne disposée sur le circuit d'arrivée d'air dans le conteneur dont l'ouverture est assujettie à la commande séquentielle.

13. Dispositif de fermentation selon l'une quelconque des revendications 9 à 12, CARACTERISE EN CE QUE le milieu solide de culture est introduit en continu dans le conteneur et les quanti-

00189

tés correspondantes de milieu fermenté en sont extraites pour permettre la réalisation de cultures continues de micro-organismes hétérogènes.

1/2                                                    0018917

Fig.1

Fig.2

0018917

Fig.3

001891

Numéro de la demande

**Office européen des brevets**

**RAPPORT DE RECHERCHE EUROPEENNE**

EP 80 40 0606

| DOCUMENTS CONSIDERES COMME PERTINENTS | | | CLASSEMENT DE LA DEMANDE (Int. Cl. 4) |
|---|---|---|---|
| Catégorie | Citation du document avec indication, en cas de besoin, des parties pertinentes | Revendica- tion concernée | |
| X | DE - C - 486 282 (F. NOLDIN)  * Page 1, ligne 51 - page 2, ligne 16; revendications 1-4; figures 1-4 *  -- | 1-5,7, 10,11 | C 12 M 1/00 |
| | FR - A - 2 090 548 (INTERMAG GE-TRANKETECHNIK A.G.)  * Revendications 1-3,6-8,13 *  -- | 1-4 | |
| A | FR - A - 2 285 454 (GIOVANOLA FRERES S.A.)  * Revendications 1,7; figures 2, 3 *  -- | 1 | **DOMAINES TECHNIQUES RECHERCHES (Int. Cl. 4)**  C 12 J 1/10 C 12 M 1/04 1/02 1/04 1/00 |
| A | DE - C - 346 460 (DEUTSCH-KOLONI-ALE GERB & FARBSTOFF GmbH)  * Revendications 1,2; exemple *  -- | 1,8 | |
| A | US - A - 2 970 088 (R.R. FREEMAN)  * Figures; revendications 1,5; colonne 1, ligne 55 - colonne 2, ligne 72 *  ---- | 1 | |

**CATEGORIE DES DOCUMENTS CITES**

X: particulièrement pertinent
A: arrière-plan technologique
O: divulgation non-ecrite
P: document intercalaire
T: théorie ou principe à la base de l'invention
E: demande faisant interférence
D: document cité dans la demande
L: document cite pour d'autres raisons

&: membre de la même famille. document correspondant

☒ Le présent rapport de recherche a été établi pour toutes les revendications

| Lieu de la recherche | Date d'achèvement de la recherche | Examinateur |
|---|---|---|
| La Haye | 11-08-1980 | COUCKE |

OEB Form 1503.1 06.78